# EUROPEAN PATENT SPECIFICATION

(11) **EP 0 389 498 B1**
(45) Date of publication and mention of the grant of the patent: **07.01.1993**
(21) Application number: 88908999.1
(22) Date of filing: 17.10.1988
(51) Int. Cl.: A61M 5/315

(54) **INJECTION SYRINGE**
SPRITZE
SERINGUE D'INJECTION

(30) Priority: 15.10.1987 NO 874306
(43) Date of publication of application: 03.10.1990
(73) Proprietor: SANDSDALEN, Christian, Malaga (ES)
(72) Inventor: SANDSDALEN, Christian, Malaga (ES)
(74) Representative: Abbie, Andrew Kenneth
(86) International application number: NO8800080
(87) International publication number: WO8903231

(56) References cited:
- EP-A- 0 229 017
- US-A- 4 391 272
- US-A- 4 687 467
- US-A- 4 699 614

## Description

The invention relates to an injection syringe for use once only and comprising a cylinder and a piston having a piston rod.

For hygienic reasons it is desirable that injection syringes are not used more than once, and in recent years simple and cheap injection syringes which are intended for use once only have been designed. However, such syringes have nevertheless often been used several times. There are also known arrangements in such syringes intended to prevent use of the syringe more than once. Thus, it is known to provide the piston and/or the piston rod with barbs which permit the piston to be moved into the cylinder only and not in the reverse direction. Such a syringe requires the piston to be filled in a factory. From EP-A-0 229 017 there is also known an arrangement in which the piston rod is connected to the piston through means which upon pressing of the piston rod against the piston during the injection break or become deformed to such an extent that it is no longer possible to retract the piston for the purpose of refilling the syringe.

Such mechanical devices depend on an accurate production and will in extreme cases, e.g. when force is supplied, not provide an absolute security against reuse.

The object of the invention is to provide a novel arrangement in injection syringes which uses means different from the previously known means for ensuring that the syringe can be used once only. According to the invention this has been achieved by producing the piston at least in part from a material which is soluble in aqueous injection media, and which it is physiologically safe to inject into the human body, said material being exposed to such a medium when the syringe is filled, whereby the piston will be dissolved and the syringe thus put out of operation. The said material can. preferably be a crystallinic glucose which can be bonded to a solid body, but which an aqueous medium contacting said body will penetrate and dissolve. The size and density of the glucose crystals, the height of the piston and the access of the injection medium to the soluble material can be so adapted as to achieve a dissolution period allowing filling and injection, but not a further injection after refilling.

The soluble material such as crystallinic glucose may conveniently be attached to the piston rod by means of rings or discs of plastic material or the like, which can also serve to provide a sealing against the cylinder wall. Such rings or discs must of course have openings in order to allow the injection medium to pentetrate into the soluble material.

The invention will now be further described, reference being made to the drawing.

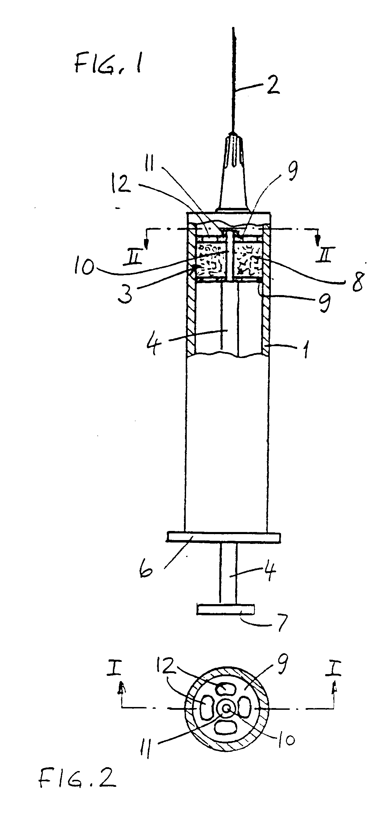

Fig. 1 illustrates - partly in section - a diagrammatical view of an injection syringe designed according to the invention.

Fig. 2 illustrates a section along the line II-II in Fig. 1.

In the drawing 1 designates a cylinder having a canula 2, whereas 3 designates a piston having a piston rod 4. The rear end of the cylinder 1 is sealed by an end wall 6 extending beyond the peripheral walls of the cylinder 1 in the usual manner. Of course, the piston rod 4 extends through the end. wall 6 and is at the end shaped with a handle 7.

The piston 3 is composed of a body 8, which according to the invention consists of a material which is soluble in aqueous injection media and which it is physiologically safe to inject into the human body, and of two plastic discs 9 on either side of the body 8, the circumference of said disc sealingly engaging the walls of the cylinder 1. The body 8 and the plastic discs 9 are mounted onto a recessed portion 10 of the piston rod 4 in abutment with a shoulder and secured on said portion 10 by means of a locking disc 11. The plastic discs 9 should have relatively large openings 12 allowing the aqueous medium to contact the body 8 and penetrate into this body when the syringe is filled.

Thereby the injection medium will dissolve the material in the body 8 so that the piston can no longer fulfil its function as a piston, the syringe thereby being put out of operation.

A suitable material for use in the body 8 is a crystallinic glucose, but also other phYsiologically safe, solid materials which are easily soluble in aqueous injection media may be used. By adapting the size and density of the crystals in the body 8 of e.g. a crystallinic glucose, as well as the height of the piston and the size of the openings 12, it becomes possible to obtain a dissolution period allowing filling and injection, but not a further injection after refilling. The period in which the piston remains in function can e.g. be adjusted to two minutes or shorter.

The discs 9 do not require any boss as indicated, but can simply consist of a ring which merely has the purpose of sealing against the cylinder wall, said ring being bonded to the body 8 in any suitable manner. The attachment of the piston to the piston rod can be obtained by letting the body 8 directly abut the shoulder of the piston rod and the locking disc 11.

## Claims

1. An injection syringe for use once only comprising a cylinder (1) and a piston (3) having a piston rod (4),
**characterized in** that the piston (3) is at least in part produced from a material which is soluble in aqueous injection media and which is physiologically safe to inject into the human body, the piston being conformed such that said material is exposed to said medium when the syringe is filled, whereby the piston (3) will be dissolved and the syringe thus put out of operation.

2. Arrangement according to claim 1,
**characterized in** that said material is crystallinic glucose.

3. Arrangement according to claim 2,
**characterized** **in** that the size and density of the glucose crystals, the height of the piston and the access of the injection medium to the soluble material are so adapted as to achieve a dissolution period allowing filling and injection, but not a further injection after refilling.

## Patentansprüche

1. Injektionsspritze zur nur einmaligen Benutzung, umfassend einen Zylinder (1) und einen Kolben (3) mit einer Kolbenstange (4),
dadurch **gekenzeichnet**, daß der Kolben (3) mindestens teilweise aus einem Material hergestellt ist, das in wäßrigem Injektionsmittel löslich und dessen Injektion in den menschlichen Körper physiologish sicher ist, wobei der Kolben so ausgelegt ist, daß das Material im gefüllten Zustand der Spritze dem besagten Mittel ausgesetzt ist, wodurch sich der Kolben (3) auflöst und so die Spritze außer Betrieb gesetzt wird.

2. Anordnung nach Anspruch 1,
dadurch **gekennzeichnet**, daß das Material kristalline Glucose ist.

3. Anordnung nach Anspruch 2,
dadurch **gekennzeichnet**, daß die Größe und die Dichte der Glucosekristalle, die Höhe des Kolbens und der Zutritt des Injektionsmittels zu dem löslichen Material so angepaßt sind, daß eine Auflösungszeit erreicht wird, die ein Füllen und eine Injektion gestattet, aber keine weitere Injektion nach erneutem Füllen.

## Revendications

1. Seringue d'injection destinée à servir une seule fois, comportant un cylindre (1) et un piston (3) muni d'une tige de piston (4), **caractérisée** en ce que le piston (3) est au moins en partie produit à partir d'une substance qui est soluble dans des milieux aqueux pour injection et physiologiquement inoffensive à injecter dans le corps humain, le piston étant conformé de façon que ladite matière soit exposée audit milieu lorsque la seringue est remplie, pour qu'ainsi le piston (3) soit dissous et la seringue par conséquent mise hors d'état de fonctionner.

2. Dispositif selon la revendication 1,**caractérisé** en ce que ladite matière est du glucose cristallisé.

3. Dispositif selon la revendication 2, **caractérisé** en ce que la taille et la densité des cristaux de glucose, la hauteur du piston et l'accès du milieu d'injection à la matière soluble sont adaptés pour obtenir une période de dissolution permettant un remplissage et une injection, mais pas d'autre injection après un nouveau remplissage.
